(19) Europäisches Patentamt
European Patent Office
Office européen des brevets

(11) **EP 3 417 774 A1**

(12) **EUROPEAN PATENT APPLICATION**

(43) Date of publication:
26.12.2018 Bulletin 2018/52

(51) Int Cl.:
*A61B 5/024* (2006.01)     *A61B 5/00* (2006.01)
*H03M 1/12* (2006.01)

(21) Application number: 18179329.0

(22) Date of filing: 22.06.2018

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 22.06.2017 US 201715629984

(71) Applicant: Infineon Technologies AG
85579 Neubiberg (DE)

(72) Inventor: FANT, Andrea
33045 Nimis (IT)

(74) Representative: Hersina, Günter
Schoppe, Zimmermann, Stöckeler
Zinkler, Schenk & Partner mbB
Patentanwälte
Radlkoferstrasse 2
81373 München (DE)

(54) **SYSTEM AND METHOD FOR MONITORING A HEART RATE**

(57)    In one embodiment, a method for monitoring a heartbeat includes receiving a heartbeat signal, the heartbeat signal including heartbeat cycles. The method also includes determining a region of interest (ROI) of the heartbeat cycles based on an identifying feature of each heartbeat cycle of the heartbeat cycles. The ROI includes a first portion of a period of a first heartbeat cycle. The method also includes sampling the first portion of the heartbeat cycle within the ROI at a first sampling rate and sampling a second portion of the heartbeat cycle outside of the ROI at a second sampling rate less than the first sample rate.

Figure 5

## Description

## TECHNICAL FIELD

The present invention relates generally to an electronic device, and, in particular embodiments, to a system and method for monitoring a heart rate.

## BACKGROUND

Wearable technology can be found, for example, in activity trackers, clothing with embedded circuits, implants, smart rings, or watches. These devices collect data from one or more sensors to provide monitoring and real time feedback to the wearer.

The introduction of miniaturized sensors packaged in a single portable device, have led to an increase in the popularity and availability of wearable devices. Persons can access and monitor an assortment of environmental and personal information with a single gesture. Companies, in order to differentiate products from competitors, have introduced devices with increasing capabilities in a reduced footprint. Manufacturers have also focused on data reliability and data accuracy as a differentiator. An elegant approach in these areas directly impact energy consumption and processing power.

An optical based heart rate sensor measures a change in light absorption on the surface of the skin in a cardiac cycle due to volumetric changes in blood in peripheral circulation. Various circuits in the electronic device are tasked with sampling and analyzing data to provide, for example, resting heart rate (HR), maximum HR, current HR, or heart rate variability (HRV). The presence of optical based heart rate sensors has steadily increased in wearable devices. As the necessary circuitry has shrunk, processing capabilities have increased, culminating in an increase in data accuracy.

## SUMMARY

In accordance with an embodiment, a method for monitoring a heartbeat includes receiving a heartbeat signal, the heartbeat signal including heartbeat cycles. The method includes determining a region of interest (ROI) of the heartbeat cycles based on an identifying feature of each heartbeat cycle of the heartbeat cycles. The ROI includes a first portion of a period of a first heartbeat cycle. The method includes sampling the first portion of the heartbeat cycle within the ROI at a first sampling rate and sampling a second portion of the heartbeat cycle outside of the ROI at a second sampling rate less than the first sample rate.

In accordance with another embodiment, a digital logic core is configured to receive a heartbeat signal comprising heartbeat cycles. The digital logic core is configured to determine a ROI of the heartbeat cycles based on an identifying feature of each heartbeat cycle of the heartbeat cycles. The ROI includes a first portion of a period of a first heartbeat cycle. The digital logic core is configured to sample portions of the heartbeat cycle within the ROI at a first sample rate, and sample portions of the heartbeat cycle outside of the ROI at a second sample rate less than the first sample rate.

In accordance with yet another embodiment, a system for monitoring heartbeat includes a light emitting diode (LED), a photodiode (PD), an analog-to-digital converter having an input converter connected to the photodiode. The digital logic circuit has an input connected to an output of the analog-to-digital converter and a clock output connected to a sampling clock input of the analog-to-digital converter and to the LED. The digital logic circuit is configured to determine a region of interest (ROI) portion of a heartbeat cycle of a heartbeat signal received by the photo diode. The digital logic circuit is further configured to provide a clock signal of a first frequency on the clock output during the ROI portion of the heartbeat cycle. The digital logic circuit is also configured to provide a clock signal of a second frequency on the clock output outside of the ROI portion of the heartbeat cycle. The first frequency is greater than the second frequency.

## BRIEF DESCRIPTION OF THE DRAWINGS

For a more complete understanding of the present invention, and the advantages thereof, reference is now made to the following descriptions taken in conjunction with the accompanying drawing, in which:

Figure 1 is a diagram of a system for heart rate monitoring;

Figure 2 is a graph of a general heartbeat signal measured by an optical system;

Figure 3 is a graph of a general heartbeat signal with sample points identified using a slow clock rate;

Figure 4 is a graph of a general heartbeat signal with regions of interest highlighted at each cycle;

Figure 5 is a graph of a general heartbeat cycle with sample points identified using a fast clock rate in the region of interest;

Figure 6 is a diagram of an optical sensor heart rate monitor circuit;

Figure 7 is another diagram of an optical sensor heart rate monitor circuit;

Figure 8 is a flowchart of an embodiment method for sampling a heartbeat at slow and fast clock rates;

Figure 9 is a block diagram of a processing system that may be used for implementing some of the devices and methods disclosed herein in accordance with embodiments of the present invention; and

Figure 10 is block diagram of a further processing system and a sensor package that may be used for implementing some of the devices and methods disclosed herein in accordance with embodiments of the present invention.

Corresponding numerals and symbols in different figures generally refer to corresponding parts unless otherwise indicated. The figures are drawn to clearly illustrate the relevant aspects of the preferred embodiments and are not necessarily drawn to scale. To more clearly illustrate certain embodiments, a letter indicating variations of the same structure, material, or process step may follow a figure number.

## DETAILED DESCRIPTION OF ILLUSTRATIVE EMBODIMENTS

The structure, manufacture, and use of embodiments are discussed in detail below. It should be appreciated, however, that this disclosure provides many applicable inventive concepts that can be embodied in a wide variety of specific contexts. The specific embodiments discussed are merely illustrative of specific ways to make and use the invention, and do not limit the scope of the invention.

Heart rate variability (HRV) is a physiological phenomenon related to a variance in the time period of one heartbeat to the next. Studies have shown that the accurate measurement of the HRV can assist in identifying health conditions such as congestive heart failure, depression, or diabetic neuropathy. An accurate sampling of a heartbeat cycle is necessary to determine the variability of the heart rate from one cycle to the next.

As health monitoring devices increase in popularity, a competitive advantage is gained by improving sensor accuracy. In such devices, with limited energy and processing resources, there is a demand to improve measurement accuracy while maintaining energy and power resource efficiency.

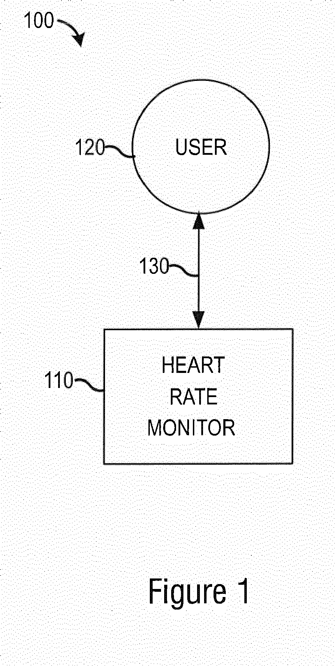

In Figure 1, a system 100 for monitoring the heart rate of a user 120 utilizing a heart rate monitoring device 110 is illustrated. Embodiments of this disclosure use an optical based heart rate monitoring (HRM) technique, such as photoplethysmography (PPG), to measure the change in light absorption on the skin tissue of the user 120 in a cardiac cycle. Generally, to achieve an accurate measurement of the variance of the heart rate from beat to beat, a relatively high temporal sampling resolution is used. This high temporal sampling resolution comes at the cost of extra power, especially since most of the power resources are invested in driving an LED in a typical system that implements optical based HRM techniques.

In an embodiment, the heart rate monitoring device 110 employs a high sampling rate system with low resolution. This maintains system power consumption within reasonable limits, while retaining sufficient time resolution (~1000 samples / second or equivalent to 1 ms time resolution) for data collection. The high sample-rate data is constantly analyzed and analytically reduced, wherein a small portion of data is extracted to calculate heart rate variability.

In embodiments where data is analyzed on a separate back-end signal processing unit (e.g. a DSP or CPU), an output bus moves the data to the processing unit whereby additional limited resources are expended. In embodiments where the system is optimized to have a low-power state for the processing unit, the data has to be then stored on the frontend device. Thus, some embodiments use a large first-in, first-out (FIFO) memory to collect enough data for the fast data stream.

In the embodiments of this disclosure, a system and method are proposed to maintain HRV measurement accuracy while reducing system power consumption. A peak prediction algorithm (time-based or frequency-based) and a variable sampling rate of the input signal are introduced, wherein the heartbeat signal is sampled at a high temporal resolution only when needed.

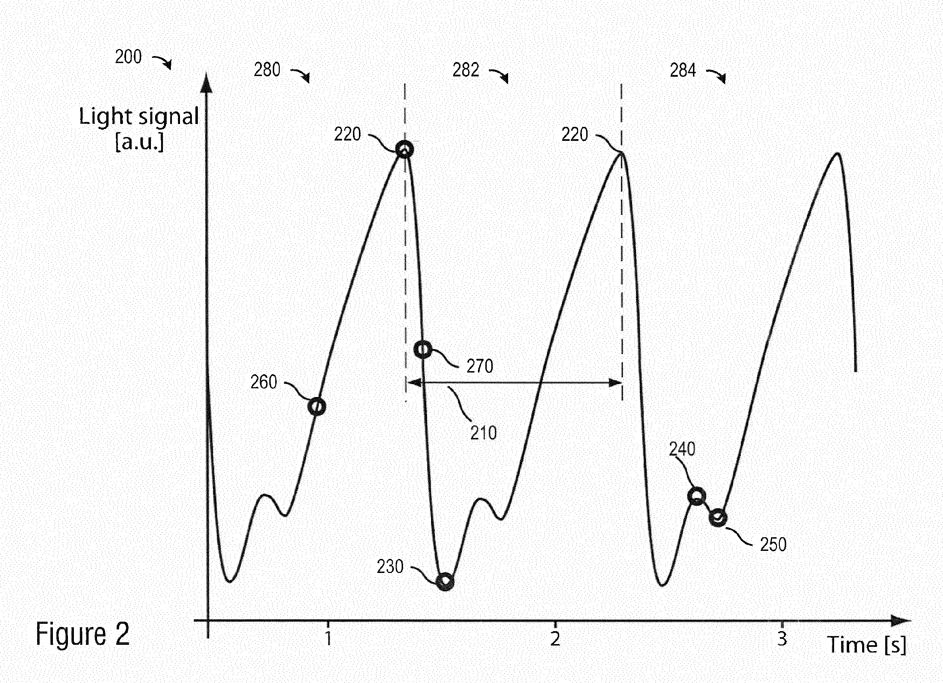

Figure 2 is a graph of a heartbeat signal 200 illustrating three heartbeat cycles 280 282 284. The period 210 for a heartbeat cycle is determined in accordance with a repeatable feature between successive heartbeat cycles. The repeatable feature can be a maximum amplitude (lowest aortic pressure point) 220, a minimum amplitude 230 (systolic peak), a dicrotic notch 240, a diastolic peak 250, a maximum positive slope after the dicrotic notch 260, a maximum negative slope 270, a sequence of multiple features, or any other repeatable feature of the heartbeat signal 200.

As an example, the heartbeat signal 200 has a period 210 of about one second, determined from maximum amplitude 220 of a first cycle 282 to maximum amplitude 220 of a second cycle 284. Alternatively, any other repeatable feature, as discussed-above, can be used to define a period 210 of a heartbeat cycle.

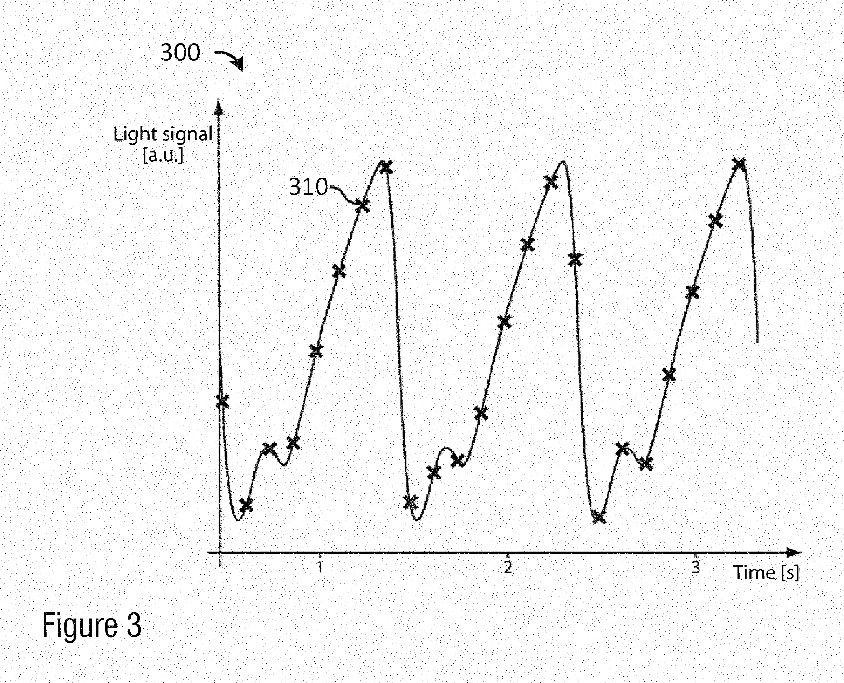

Figure 3 is a graph of a heartbeat signal 300, sampled at a constant sampling rate across the entire signal, as may be performed by a heart rate monitoring device 110. The heart rate monitor 110 samples the heartbeat signal 300 at a at a low rate with sufficient detail to collect the shape of the analog signal and recognize the position of the maximum amplitude 220, minimum amplitude 230, or any other repetitive feature of the heartbeat signal 300. The sampling points 310 are marked with crosses on the heartbeat signal 300.

In accordance with an embodiment of the present disclosure, the heart rate monitor 110 samples the heartbeat signal 300 at a first clock rate across a sequence of heartbeat cycles. In some cases, this first clock rate is slow enough that the limited data collected in this initial step does not provide enough information to accurately measure the variability of the heart rate from one cycle to the next, across the sequence of heartbeats.

In collecting the limited sample set, the heart rate monitor 110 makes a general outline of the heartbeat signal 300 across multiple heartbeat cycles. A variety of techniques can be utilized to identify a region within a heartbeat cycle that contains a repeatable feature of the heartbeat signal 300. As an example, if the slope among three sequential data points suddenly changes from a negative to a positive or from a positive to negative slope, a valley or peak is respectively determined.

The samples collected at the first clock rate include information to make an approximate determination of the location of the identifiable feature of the heartbeat signal 300. As an example, the signal in Figure 3 is sampled at a clock rate of 8 times per second. The first clock rate may be increased to improve the approximation in the determination. Alternatively, the first clock rate may be reduced to improve efficiency in terms of processing power and energy resources. A higher first clock rate, such as 32 or 64 samples per second, may be used for higher heartbeat rates, for example during or just after a period of activity or exercise; a lower, such as 8 or 16 samples per second, one would be sufficient when the user 120 is at rest or during sleep.

Although a variation exists in the period of a heartbeat, in a sequence of heartbeat signals, the period from one cycle to the next might not change significantly. This characteristic provides an opportunity to estimate the location of the repeatable feature within a reasonable region for future heartbeat cycles based on information collected at previous heartbeat cycles.

In response to the determination of the location of an identifiable feature of a heartbeat signal 300, a region of interest (ROI) that contains the estimated location is identified. This ROI provides guidance to the heart rate monitoring device 110 to increase the sampling in the ROI to more accurately identify the location of the heartbeat signals maximum amplitude 220, minimum amplitude 230, or any other identifiable feature. Additionally, the width of the ROI is large enough to contain any major shift in the peak or valley from one cycle to the next.

In some embodiments, the heart rate monitoring device 110 measures the location in time of the identifiable feature of a heartbeat cycle. The increase in sampling rate allows for greater fidelity of the measurements. The heart rate monitoring device 110 calculates the difference in time, beat-to-beat interval, between a measured identifiable feature in one heartbeat cycle to an adjacent cycle in the heartbeat signal 300. The HRV is a measurement of the variation of the beat-to-beat interval.

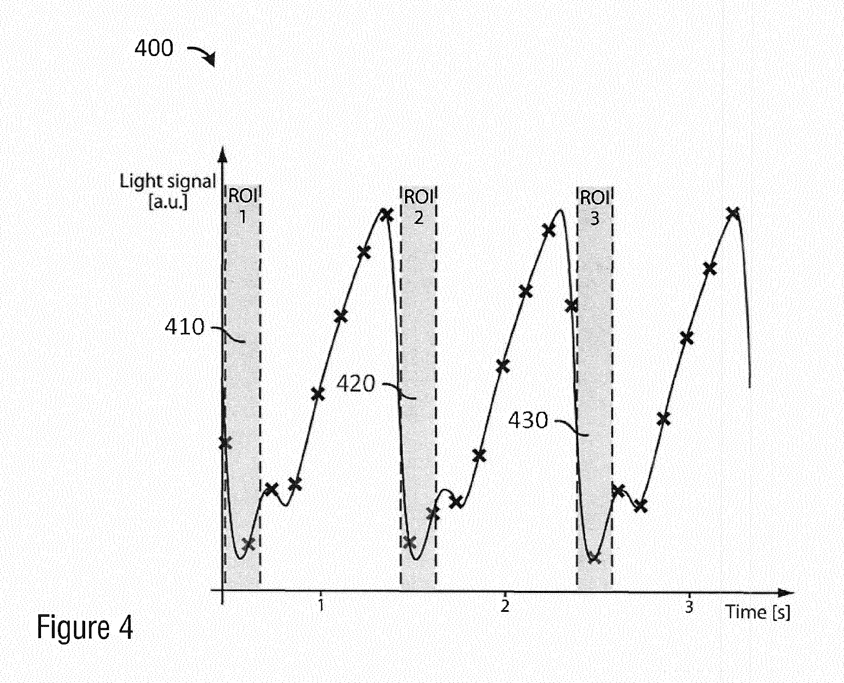

Figure 4 is a graph of a heartbeat signal 400 illustrating a sequence of ROIs 410-430 corresponding to each heartbeat cycle in accordance with a constant sampling rate. As previously disclosed and illustrated in Figure 4, one of the identifiable features of a heartbeat signal 300 is a minimum amplitude 230 determined by a change in slope from a negative direction to a positive direction. The ROIs 410-430 of each heartbeat cycle contain at least one sampled point corresponding to the valley of each heartbeat. A ROI can contain multiple sample points as illustrated in ROI 420.

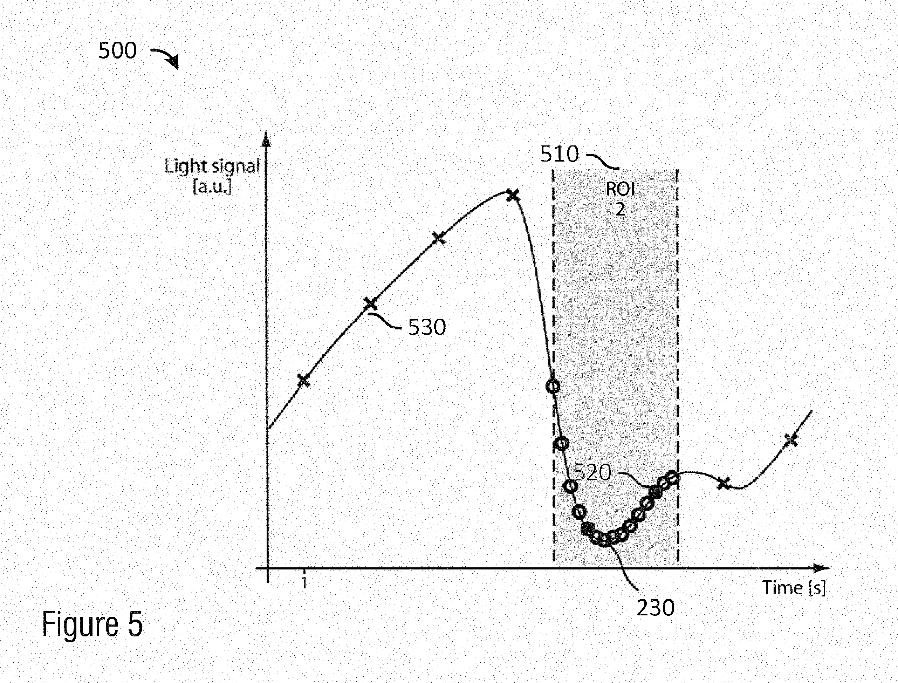

Figure 5 is a graph of a heartbeat cycle 500 illustrating a ROI 510 sampled at a fast clock rate (64 samples / second) and a region outside of the ROI 510 sampled at a slow clock rate (8 samples / second). In the ROI 510, the sampling points are illustrated as circles 520. Outside the ROI 510, the sampling points are illustrated by crosses 530. The ROI 510 in Figure 5 corresponds to the ROI 420 in Figure 4. The increased sampling in the ROI enables a high temporal resolution in an area of the heartbeat cycle 500 that contains a minimum amplitude 230. The accuracy of the HRV from one cycle to the next is directly correlated with the accuracy in determining the repeatable identifiable feature of the heartbeat cycle 500. The high temporal resolution in the ROI 510 provides this information to the heart rate device monitor 110 while reducing the sample points, improving power efficiency, reducing latency, and overhead related to the analysis of an unnecessarily large data sample outside of the ROI 510. The process of determining and sampling the ROI 510 at a high temporal resolution is repeated at successive heartbeat cycles, providing more accurate HRV measurements at a reduced power overhead. In general, the system can continuously update the ROI by analyzing the collected sampled data. The ROI can also be adjusted cycle by cycle to track the position of the minimum amplitude 230 - or one or more identifiable feature - with a high resolution.

In an embodiment, the width of the ROI 510 can be adjusted to reduce or minimize the region with high temporal resolution, based on the currently measured HRV. The reduction in duration of the high temporal resolution region reduces the size of the collected data. This reduction in data size improves efficiency and reduces the extra overhead related to analyzing data points outside of the ROI 510. The position of the ROI 510 with respect to the heartbeat cycle 500 can be shifted around the identifying feature of the heartbeat cycle 500. In an embodiment, the position of the ROI 510 is shifted to improve the detection of the period of the heartbeat cycle 500. In another embodiment, the position of the ROI 510 can be positioned in a region with a maximum positive slope, maximum negative slope, or the like, for an improved detection of the signal period. In the regions outside of the ROI 510, the slow sample rate enables other functions such as baseline removal and artifact compensation.

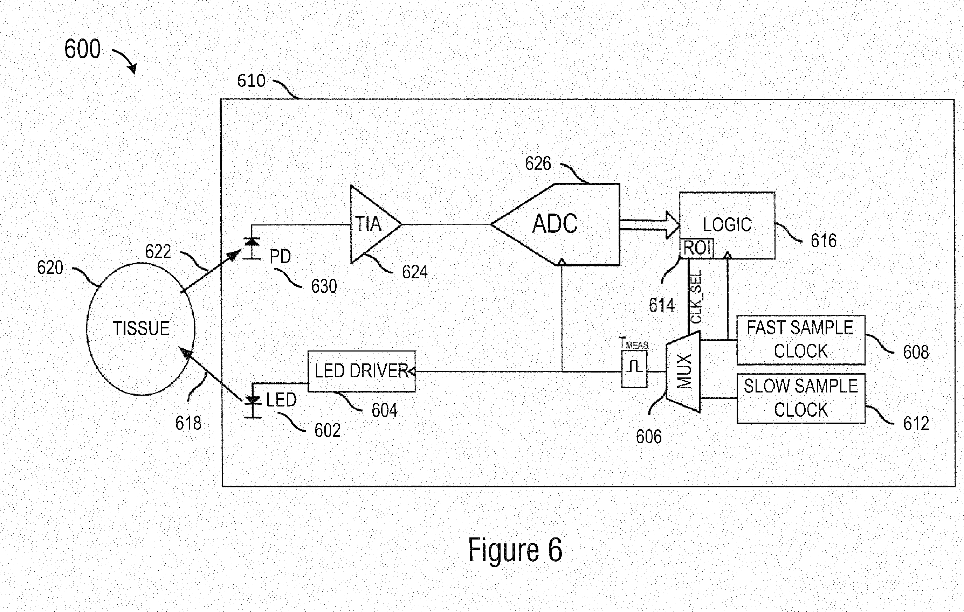

Figure 6 illustrates a block diagram of a system 600 according to an embodiment that includes an optical sensor heart rate monitoring device 610 that implements a method of HRV sampling. The heart rate monitoring device 610 uses PPG to measure changes in light absorption on the skin tissue 620 in a cardiac cycle. The heart rate monitoring device 610 is comprised of various components that sample and analyze a heartbeat signal 400. In the heart rate monitoring device 610, a light emitting diode 602 (LED) is coupled to a LED driver 604. A multiplexer 606 is coupled to a region of interest (ROI) circuit block 614 located in the digital logic core 616. The multiplexer 606 toggles a sample clock, between a fast 608 and a slow clock rate 612. The ROI circuit block 614 determines a variable sample rate and directs the multiplexer 606 to sample the heartbeat signal 400 accordingly.

The LED 602 generates light waves 618 that illuminate the surface of the skin tissue 620. The system may have one or more LEDs 602. A photodiode 630 (PD) monitors the pressure pulse by measuring the transmitted or reflected light wave 622 from the surface of the skin tissue 620. The PD 630 generates an analog electrical current representation of the pressure pulse as received in the form of an optical energy signal. The system may have one or more PDs 630.

The TIA 624 converts the electrical current produced by the PD 630 to an analog voltage signal. The analog voltage is then converted, by an analog-to-digital converter 626 (ADC) to a digital signal.

In accordance with embodiment methods described herein, the digital logic core 616, taking into account the source clock rate, processes the digital signal. The digital logic core 616 can be implemented as a microprocessor, a microcontroller, an application specific integrated circuit (ASIC), a digital signal processor, a state machine, a custom logic circuit, a field programmable gate array (FPGA) or the like.

As previously described, once a heartbeat is roughly characterized and a ROI has been identified, subsequent heartbeat samples are collected by illuminating the skin at a fast clock rate in the ROI 510 and a slow clock rate in the region outside of the ROI 510. This is done by controlling the sample rate at the multiplexer 606 coupled to the fast clock source 608 and the slow clock source 612. The multiplexer 606 receives a control signal by the ROI block 614 in the digital core 616.

In some embodiments, each component may be implemented as a separate chip or each component may be implemented in a single microcontroller or ASIC. In other embodiments, each component may receive various other clock signals or some components may be implemented as asynchronous digital logic. In alternative embodiments, the various components in the heart rate monitoring device 610 may be implemented as analog circuits.

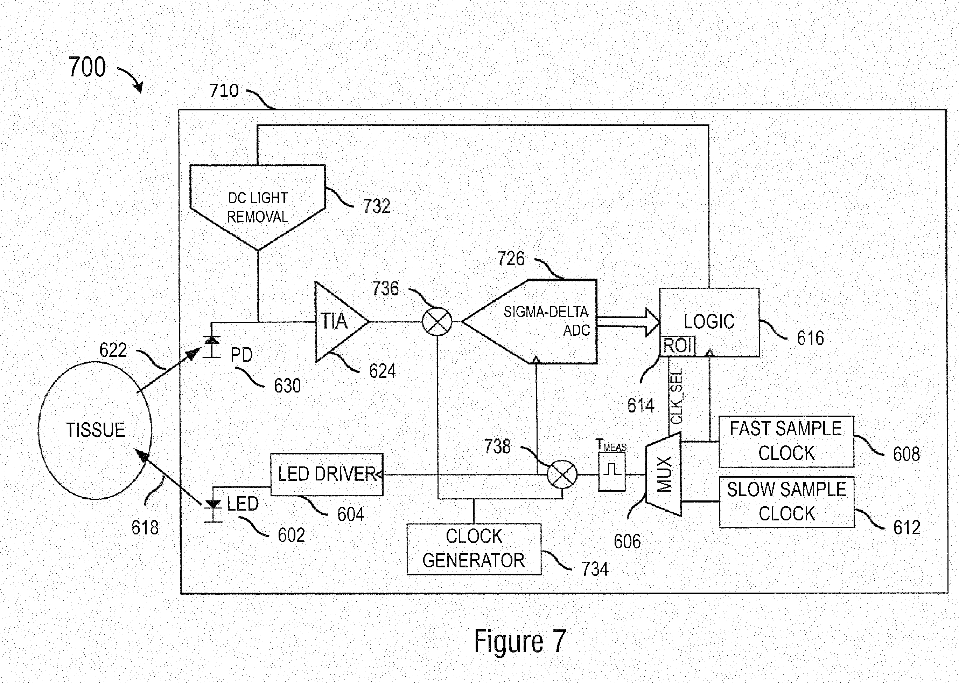

Figure 7 illustrates a block diagram of an embodiment heartbeat monitoring system 700 in which the input to the LED 602 is modulated and the output of the PD 630 is demodulated prior to sampling the transmitted or reflected light wave 622 at the digital logic core 616. As shown, modulator 738 is coupled between multiplexer 606 and LED driver 604 and demodulator 736 is coupled between trans-impedance amplifier 624 and ADC 726. During operation, modulator 738 modulates the clocked signal provided by multiplexer 606 with the output of clock generator 734 to produce a modulated signal. This modulated signal is used by LED driver 604 to produce modulated light 618 from LED 602. Reflected modulated light 622 is detected by PD 630 and amplified by trans-impedance amplifier 624 to produce an amplified modulated reflected signal. This amplified modulated reflected signal is demodulated by demodulator 736 according to the clock signal generated by the clock generator 734, and digitized by ADC 726.

The modulator 738 and the corresponding demodulator 736 may be realized, for example, using a chopper modulator and a chopper demodulator circuit. In an embodiment, the chopper circuit modulates the clocked signal with an ON/OFF behavior in a synchronized manner. The clock generator 734 synchronizes the modulator 738 and the demodulator 736.

The demodulator 736, in turn, demodulates the signal by un-doing the chopping process. In an embodiment, the demodulator 736 may be a double sampler circuit. In this embodiment, the double sampler circuit subtracts the signal amplitude when the LED is ON from the signal amplitude when the LED is OFF.

In an embodiment, the ADC 726 may be running at a rate corresponding to the demodulator 736. As an example, when the demodulator 736 is a double sampler circuit, the ADC 726 may be running at half the rate of the demodulator 736 due to the double sampling operation of the demodulator 736.

In an embodiment, the ADC 726 may be an incremental sigma delta ADC where the ADC 726 is integrating during the modulation period to collect a single sample. The signal path is effectively turned OFF in between the collection of the samples. In this mode, the output rate may not be directly related to the oversampling ratio as is typical in a regular sigma delta converter.

As shown in figure 7, the ADC 626 of figure 6 may be implemented as a sigma-delta ADC 726. In alternative embodiments, other ADC architectures known in the art may be used.

In some embodiments, DC light removal block 732 may be used to eliminate saturation at the TIA 624 owing to the effects of the existing ambient light during measurement. The DC light removal block 732 removes the DC component of the heartbeat signal such that only the AC component of the signal is received at the TIA 624. As an example, the DC light removal block 732 may be realized by a feedback loop having a circuit that tracks the mean value of the input signal and generates a corresponding DC offset correction signal. The DC offset correction signal may then be subtracted from the input signal to generate an adjusted input signal. Similar methods may be used that are known in the art.

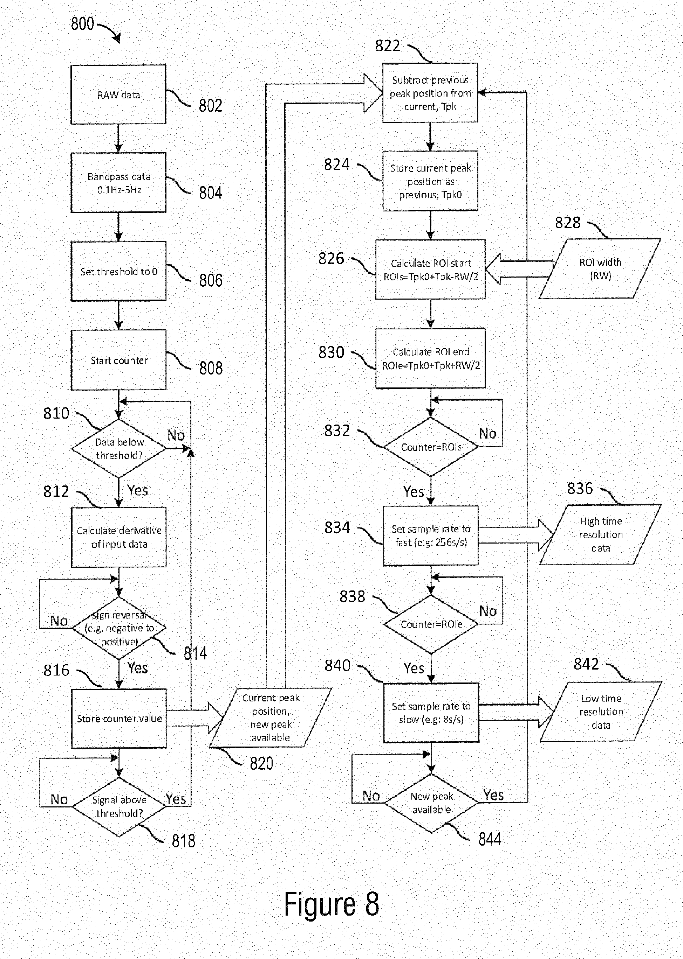

Figure 8 is a flowchart of an embodiment method 800 for sampling a heartbeat signal 400 as performed by the optical heart rate monitoring device 610. In the embodiment of the system 600, an algorithm uses the minimum amplitude 230 corresponding to an aortal maximum pressure as a repetitive feature to track. At step 802, the monitoring device 110 receives the raw data. At step 804, the algorithm applies a bandpass filter to the raw data. At step 806, the high-pass component of the filter centers the resulting data to zero and is set as a threshold. At step 808, a sequence to identify the repetitive feature of the heartbeat signal 400 begins at the zero data point with a counter equal to zero. At a point where the filtered data falls below a zero level, at step 810, the derivative of the filtered data is calculated, step 812. At step 814, the change of sign of the derivative identifies the negative minimum of the waveform, corresponding to the systolic peak of the aortal blood pressure. When the repeatable feature is identified, the value of the counter is stored at step 816 and the system flags the event for the ROI calculation process at step 820. The system waits for the signal to go back above the threshold at step 818. Once the signal goes above the threshold, the loop restarts from step 810. Other methods could include the maximum of the second derivative, or any other peak finding method in time domain.

Once the peak is found, at step 820, the position is passed to the ROI calculation process. In the ROI calculation process, the position of the peak is compared to the position of the last recorded peak. A time differential (Tpk) between the two values is computed, at step 822, to determine the signal period to calculate the center of the next ROI. The current peak position (Tpko) is stored as the last recorded peak position for the next cycle, at step 824.

At step 826, the starting position for the ROI (ROIs) and at step 830, the ending position for the ROI (ROIe) are calculated using the parameter ROI Width (RW), step 828, which could be constant or adaptive based on the current HRV value. As previously mentioned, the RW can be adjusted to reduce or minimize the region with high temporal resolution, based on the currently measured HRV. The starting position for the ROI (ROIs) is given by the following equation:

$$ROIs = Tpk + Tpk0 - RW/2.$$

The ending position for the ROI (ROIe) is given by the following equation:

$$ROIe = Tpk + Tpk0 + RW/2.$$

At step 832, and at the beginning of the ROIs, the algorithm changes the sampling rate, generating the high temporal resolution data stream, step 834, within the ROI that will be used for the accurate HRV extraction, step 836. At step 838, when the algorithm encounters the end the of the ROI 510, the sampling rate is changed, step 840, to the slow rate to measure the low temporal resolution data stream, step 842. The process is repeated for the successive cycle in a heartbeat signal, step 844.

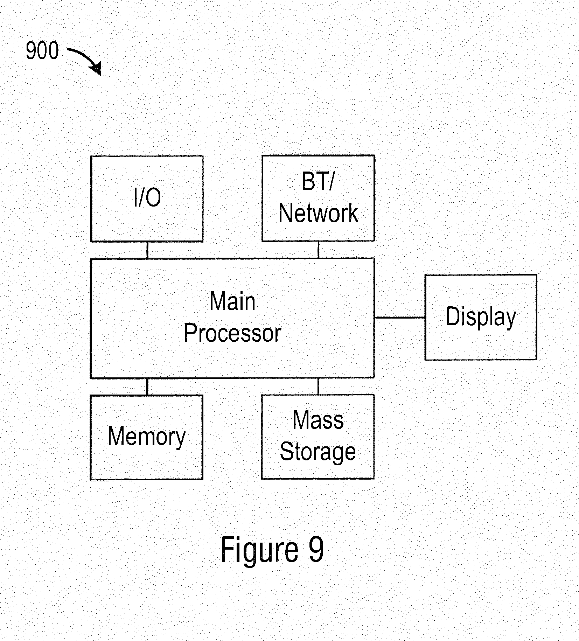

Figure 9 shows a block diagram of a processing system 900 that may be used for implementing some of the devices and methods disclosed herein. Specific devices may utilize all of the components shown, or only a subset of the components, and levels of integration may vary from device to device. Furthermore, a device may contain multiple instances of a component, such as multiple processors, displays, memories, etc. The processing system may comprise a processing unit equipped with one or more input/output devices, such as a speaker, microphone, mouse, touchscreen, keypad, keyboard, printer, display, and the like. The processing unit may include a CPU, memory, a mass storage device, a display, and an I/O interface connected to a bus. In an embodiment, multiple processing units in a single processing system or in multiple processing systems may form a distributed processing pool or distributed editing pool.

A person of skill in the art would readily recognize that acts of various above-described methods may also be performed by microprocessors external to the heart rate monitoring device. In these embodiments, data may be distributed directly to the microprocessor or may be stored in an embodiment in a storage device. Herein, some example embodiments are also intended to cover program storage devices, e.g., digital data storage media, which are machine or computer readable and encode machine-executable or computer-executable programs of instructions, wherein the instructions perform some or all of the acts of the above-described methods. The program storage devices may be, e.g., digital memories, magnetic storage media such as magnetic disks, and magnetic tapes, hard drives, or optically readable digital data storage media.

The bus may be one or more of any type of several bus architectures including a memory bus or memory controller, a peripheral bus, video bus, or the like. The CPU may comprise any type of electronic data processor. The memory may comprise any type of system memory such as random access memory (RAM), static RAM (SRAM), dynamic RAM (DRAM), synchronous DRAM (SDRAM), read-only memory (ROM), a combination thereof, or the like. In an embodiment, the memory may include ROM for use at boot-up, and DRAM for program and data storage for use while executing programs.

The mass storage device may comprise any type of storage device configured to store data, programs, and other information and to make the data, programs, and other information accessible via the bus. The mass storage device may comprise, for example, one or more of a solid state drive, hard disk drive, a magnetic disk drive, an optical disk drive, or the like.

The display and the I/O interface provide interfaces to couple external input and output devices to the processing unit. Examples of input and output devices include the display coupled to a video adapter and the mouse/keyboard/printer coupled to the I/O interface. Other devices may be coupled to the processing unit, and additional or fewer interface cards may be utilized. For example, a serial interface such as Universal Serial Bus (USB) (not shown) may be used to provide an interface for a printer.

The processing unit also includes one or more network interfaces, which may comprise wired links, such as an Ethernet cable or the like, and/or wireless links to access nodes or different networks. The network interface allows the processing unit to communicate with remote units via the networks. For example, the network interface may provide wireless communication via one or more transmitters/transmit antennas and one or more receivers/receive antennas. In an embodiment, the processing unit is coupled to a local-area network or a wide-area network for data processing and communications with remote devices, such as other processing units, the Internet, remote storage facilities, or the like. The network interface may be configured to have various connection-specific virtual or physical ports communicatively coupled to one or more of these remote devices.

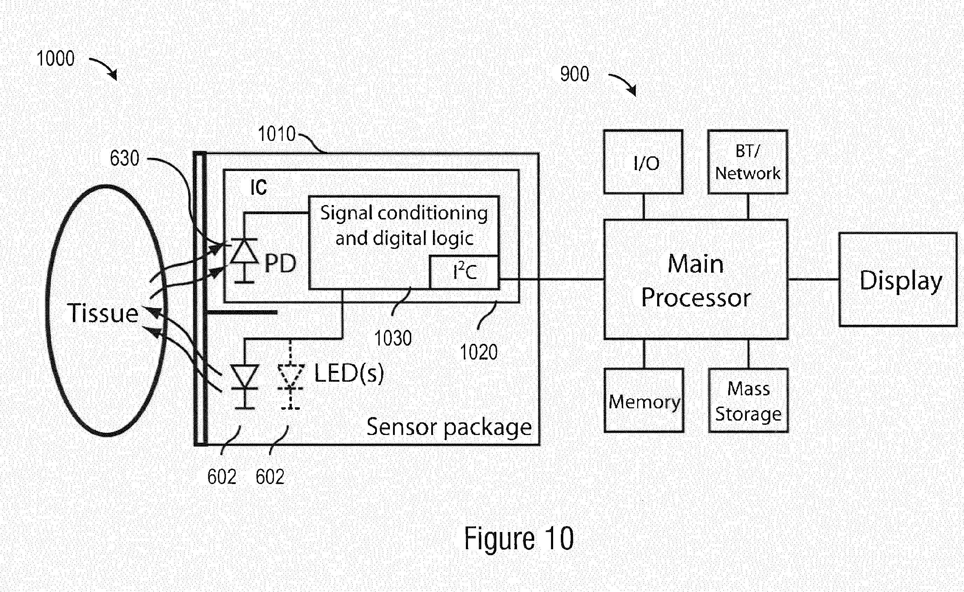

Figure 10 is a block diagram illustrating an embodiment heart rate monitoring system 1000 of the processing system 900 illustrated in Figure 9, a heart rate monitoring device 610 as illustrated in Figure 6, and packaged in a sensor package 1010 as illustrated in Figure 10. The sensor package 1010 may include one or more LEDs 602 and an integrated circuit 1020 (IC). The IC 1020 may include the logic core and one or more PDs 630 in the heart rate monitoring device 610. The sensor package 1010 is coupled to the processing system 900 which may or may not be part of the sensor package 1010.

The IC 1020 may contain the signal condition components such as the various clock sources 608 612 734, modulator 736, demodulator 738, multiplexer 606, and ADCs 626 726 as illustrated in Figures 6 and 7. Some of these components may be located outside of the IC 1020. The IC 1020 may contain both signal conditioning components and the digital logic core as illustrated in figures 6 and 7. In various embodiments, IC 1020 is implemented on a single semiconductor substrate, such as a silicon substrate. In some embodiments, the IC 1020 may be an ASIC.

A system such as illustrated in Figure 10 may be integrated with a great liberty of arrangement of blocks 1010 and 900. In an embodiment, the processing system 900 may be packaged as a system with the sensor package 1010. In another embodiment, one or more components of the processing system 900 may receive the data from the sensor package 1010 at a separate location such as a standalone computer or a remote processing unit such as a cloud network.

In an embodiment, the processing system 900 and the sensor package 1010 may communicate with each other by a connected bus. The data may be transferred over a communication protocol such as an inter-integrated circuit (I2C) protocol, or the like.

Example embodiments may further provide an executable program having a program code for performing one of the above methods, when the executable program is executed on a microprocessor or logic core.

Example embodiments of the present invention are summarized here. Other embodiments can also be understood from the entirety of the specification and the claims filed herein.

Example 1: A method of monitoring a heartbeat includes receiving a heartbeat signal having heartbeat cycles. The method also includes determining a region of interest (ROI) of the heartbeat cycles based on an identifying feature of each heartbeat cycle of the heartbeat cycles. The ROI is a first portion of a period of a first heartbeat cycle. The method further includes sampling the first portion of the heartbeat cycle within the ROI at a first sampling rate and sampling a second portion of the heartbeat cycle outside of the ROI at a second sampling rate, such that the second sampling rate is less than the first sampling rate.

Example 2: The method of example 1, where the determining the ROI further includes determining the identifying feature of each heartbeat cycle.

Example 3: The method of one of examples 1 and 2, where the identifying feature of each heartbeat cycle is one or more of a maximum amplitude, a minimum amplitude, a maximum positive slope, and a maximum negative slope.

Example 4: The method of one of examples 1 and 2, where the identifying feature of each heartbeat cycle is one or more of a lowest aortic pressure point, a systolic peak, a dicrotic notch, or a diastolic peak.

Example 5: The method of one of examples 1 to 4, further including calculating a difference in time between an identifying feature of a first heartbeat cycle and an identifying feature of a second heartbeat cycle in the heartbeat signal, where the first heartbeat cycle and second heartbeat cycle are sequential.

Example 6: The method of one of examples 1 to 5, where the first sampling rate is greater than or equal to 64 samples per second and less than or equal to 1000 samples per second.

Example 7: A digital logic core configured to receive a heartbeat signal having heartbeat cycles. The digital logic core further configured to determine a region of interest (ROI) of the heartbeat cycles based on an identifying feature of each heartbeat cycle of the heartbeat cycles. The ROI is a first portion of a period of a first heartbeat cycle. The digital logic core is also configured to sample portions of the heartbeat cycle within the ROI at a first sample rate and sample portions of the heartbeat cycle outside of the ROI at a second sample rate, such that the second sample rate is less than the first sample rate.

Example 8: The digital logic core of example 7, further configured to determine the identifying feature of each heartbeat cycle of the heartbeat cycles.

Example 9: The digital logic core of one of examples 7 and 8, further configured to calculate a start of the ROI using at least one of an adaptive or a constant width corresponding to the ROI.

Example 10: The digital logic core of one of examples 7 to 9, further configured to calculate an end of the ROI using the adaptive or constant width corresponding to the ROI.

Example 11: The digital logic core of one of examples 7 to 10, further including an ROI circuit block configured to direct a multiplexer to toggle between the first sample rate and the second sample rate in accordance with the ROI.

Example 12: The digital logic core of one of examples 7 to 11, further configured to calculate a difference in time between an identifying feature of a first heartbeat cycle and an identifying feature of a second heartbeat cycle in the heartbeat signal, where the first heartbeat cycle and second heartbeat cycle are sequential.

Example 13: A system for monitoring heartbeat including a light emitting diode (LED), a photodiode (PD), an analog-to-digital converter (ADC), and a digital logic circuit. The ADC having an input converter coupled to the PD and an output coupled to an input of the digital logic circuit. The digital logic circuit having a clock output coupled to a sampling clock input of the analog-to-digital converter and to the LED. The digital logic circuit is configured to determine a region of interest (ROI) portion of a heartbeat cycle of a heartbeat signal received by the PD. The digital logic circuit is further configured to provide a clock signal of a first frequency on the clock output during the ROI portion of the heartbeat cycle. The digital logic circuit is also configured to provide a clock signal of a second frequency on the clock output outside of the ROI portion of the heartbeat cycle, where the first frequency is greater than the second frequency.

Example 14: The system of example 13, further including a trans-impedance amplifier coupled between the PD and the ADC.

Example 15: The system of one of examples 13 and 14, further including a modulator and a demodulator.

The modulator is coupled between the clock output of the digital logic circuit and the demodulator is coupled between the PD and the input of the ADC.

Example 16: The system of one of examples 13 to 15, where the digital logic circuit includes a multiplexer, a first clock generator, and a second clock generator. The multiplexer having an output coupled to the clock output of the digital logic circuit. The first clock generator having an output coupled to a first input of the multiplexer and where the first clock generator is configured to produce the clock signal of the first frequency. The second clock generator having an output coupled to a second input of the multiplexer and the second clock generator is configured to produce the clock signal of the second frequency.

Example 17: The system of one of examples 13 to 16, where the ADC, the PD, and the digital logic circuit are integrated on a single semiconductor substrate.

Example 18: The system of one of examples 13 to 17, where the LED and the single semiconductor substrate are integrated in a single package.

Example 19: The system of one of examples 13 to 18, where the digital logic circuit is further configured to determine the ROI portion of the heartbeat cycle by determining an identifying feature of the heartbeat cycle.

Example 20: The system of one of examples 13 to 19, where the identifying feature of the heartbeat cycle is a lowest aortic pressure point, a systolic peak, a dicrotic notch, or a diastolic peak.

Example 21: The system of one of examples 13 and 20, where the digital logic circuit is further configured to calculate a difference in time between an identifying feature of a first heartbeat cycle and an identifying feature of a second heartbeat cycle in the heartbeat signal, where the first heartbeat cycle and second heartbeat cycle are sequential.

While this invention has been described with reference to illustrative embodiments, this description is not intended to be construed in a limiting sense. Various modifications and combinations of the illustrative embodiments, as well as other embodiments of the invention, will be apparent to persons skilled in the art upon reference to the description. It is therefore intended that the appended claims encompass any such modifications or embodiments.

**Claims**

1. A method of monitoring a heartbeat, the method comprising:

   receiving a heartbeat signal, the heartbeat signal comprising heartbeat cycles;
   determining a region of interest (ROI) of the heartbeat cycles based on an identifying feature of each heartbeat cycle of the heartbeat cycles, the ROI comprising a first portion of a period of a first heartbeat cycle;
   sampling the first portion of the heartbeat cycle within the ROI at a first sampling rate; and
   sampling a second portion of the heartbeat cycle outside of the ROI at a second sampling rate less than the first sampling rate.

2. The method of claim 1, wherein determining the ROI further comprises determining the identifying feature of each heartbeat cycle,
   wherein the identifying feature of each heartbeat cycle is one or more of a maximum amplitude, a minimum amplitude, a maximum positive slope, and a maximum negative slope, or
   wherein the identifying feature of each heartbeat cycle is one or more of a lowest aortic pressure point, a systolic peak, a dicrotic notch, or a diastolic peak.

3. The method of claim 1 or 2, further comprising calculating a difference in time between an identifying feature of a first heartbeat cycle and an identifying feature of a second heartbeat cycle in the heartbeat signal, wherein the first heartbeat cycle and second heartbeat cycle are sequential.

4. The method of any of claims 1 to 3, wherein the first sampling rate is greater than or equal to 64 samples per second and less than or equal to 1000 samples per second.

5. A digital logic core configured to:

   receive a heartbeat signal comprising heartbeat cycles;
   determine a region of interest (ROI) of the heartbeat cycles based on an identifying feature of each heartbeat cycle of the heartbeat cycles, the ROI comprises a first portion of a period of a first heartbeat cycle;
   sample portions of the heartbeat cycle within the ROI at a first sample rate; and
   sample portions of the heartbeat cycle outside of the ROI at a second sample rate less than the first sample rate.

6. The digital logic core of claim 5, further configured to determine the identifying feature of each heartbeat cycle of the heartbeat cycles, and/or
   further configured to calculate a difference in time between an identifying feature of a first heartbeat cycle and an identifying feature of a second heartbeat cycle in the heartbeat signal, wherein the first heartbeat cycle and second heartbeat cycle are sequential.

7. The digital logic core of claim 7 or 8, further configured to calculate a start of the ROI using at least one of an adaptive or a constant width corresponding to the ROI, and/or
   further configured to calculate an end of the ROI using the adaptive or constant width corresponding to the ROI.

8. The digital logic core of any of claims 5 to 7, further comprising an ROI circuit block configured to direct a multiplexer to toggle between the first sample rate and the second sample rate in accordance with the ROI.

9. A system for monitoring heartbeat, the system comprising:

   a light emitting diode (LED);
   a photodiode (PD);
   an analog-to-digital converter (ADC) having an input converter coupled to the PD; and
   a digital logic circuit having an input coupled to an output of the ADC and a clock output coupled to a sampling clock input of the ADC and to the LED, the digital logic circuit configured to:

      determine a region of interest (ROI) portion of a heartbeat cycle of a heartbeat signal received by the PD,
      provide a clock signal of a first frequency on the clock output during the ROI portion of the heartbeat cycle, and
      provide a clock signal of a second frequency on the clock output outside of the ROI portion of the heartbeat cycle, wherein the first frequency is greater than the second frequency.

10. The system of claim 9, further comprising a transimpedance amplifier coupled between the PD and the ADC.

11. The system of claim 9 or 10, further comprising:

    a modulator coupled between the clock output of the digital logic circuit; and
    a demodulator coupled between the PD and the input of the ADC.

12. The system of any of claims 9 to 11, wherein the digital logic circuit comprises:

　　a multiplexer having an output coupled to the clock output;
　　a first clock generator having an output coupled to a first input of the multiplexer, the first clock generator configured to produce the clock signal of the first frequency; and
　　a second clock generator having an output coupled to a second input of the multiplexer, the second clock generator configured to produce the clock signal of the second frequency.

13. The system of any of claims 9 to 12, wherein the ADC, the PD, and the digital logic circuit are integrated on a single semiconductor substrate, and/or wherein the LED and the single semiconductor substrate are integrated in a single package.

14. The system of any of claims 9 to 13, wherein the digital logic circuit is further configured to determine the ROI portion of the heartbeat cycle by determining an identifying feature of the heartbeat cycle, and wherein the identifying feature of the heartbeat cycle is a lowest aortic pressure point, a systolic peak, a dicrotic notch, or a diastolic peak.

15. The system of any of claims 9 to 14, wherein the digital logic circuit is further configured to calculate a difference in time between an identifying feature of a first heartbeat cycle and an identifying feature of a second heartbeat cycle in the heartbeat signal, wherein the first heartbeat cycle and second heartbeat cycle are sequential.

Figure 1

Figure 2

Figure 3

EP 3 417 774 A1

Figure 4

Figure 5

EP 3 417 774 A1

Figure 6

Figure 7

EP 3 417 774 A1

Figure 8

900

I/O

BT/
Network

Main
Processor

Display

Memory

Mass
Storage

Figure 9

900

Display

BT/ Network

I/O

Main Processor

Mass Storage

Memory

1000

630

Tissue

IC

1010

PD

Signal conditioning and digital logic

I²C

1020

1030

LED(s)

602 602

602

Sensor package

Figure 10

Europäisches
Patentamt

European
Patent Office

Office européen
des brevets

# EUROPEAN SEARCH REPORT

Application Number

EP 18 17 9329

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/213267 A1 (LAAKKONEN HARRI MATTI-HEIKKI [FI] ET AL) 28 July 2016 (2016-07-28) * paragraph [0052] - paragraph [0062] * * paragraph [0090] - paragraph [0109] * * paragraph [0003] * * paragraph [0077] - paragraph [0087] * * figures * | 1-6 | INV. A61B5/024 A61B5/00 H03M1/12 |
| X | US 2013/324809 A1 (LISOGURSKI DANIEL [US] ET AL) 5 December 2013 (2013-12-05) | 1,2,5,7, 9-12,14, 15 | |
| Y | * paragraph [0153] - paragraph [0155] * * paragraph [0162] - paragraph [0167] * * paragraph [0062] - paragraph [0070] * * paragraph [0094] - paragraph [0100] * * paragraph [0075] - paragraph [0082] * * figures 1,3,17,18 * | 13 | |
| X | US 2016/143566 A1 (BALLAM ROBERT SCOTT [AU] ET AL) 26 May 2016 (2016-05-26) * paragraph [0026] - paragraph [0033] * * paragraph [0044] - paragraph [0058] * * figures 1-7 * | 1-3,5,6 | TECHNICAL FIELDS SEARCHED (IPC) A61B H03M |
| X | EP 2 294 978 A1 (IMEC [BE]) 16 March 2011 (2011-03-16) | 1,8 | |
| A | * paragraph [0035] - paragraph [0037] * * paragraph [0042] - paragraph [0045] * * paragraph [0060] - paragraph [0070] * * paragraph [0076] * * figures 2,3,5,8-10 * | 4,12,13 | |
| Y | US 2014/275854 A1 (VENKATRAMAN SUBRAMANIAM [US] ET AL) 18 September 2014 (2014-09-18) | 13 | |
| A | * paragraph [0131] - paragraph [0135] * * paragraphs [0245], [0295] * | 10,11 | |

The present search report has been drawn up for all claims

| Place of search | Date of completion of the search | Examiner |
|---|---|---|
| The Hague | 22 October 2018 | Görlach, Tobias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 17 9329

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-2018

| Patent document cited in search report | | Publication date | Patent family member(s) | | Publication date |
|---|---|---|---|---|---|
| US 2016213267 | A1 | 28-07-2016 | NONE | | |
| US 2013324809 | A1 | 05-12-2013 | NONE | | |
| US 2016143566 | A1 | 26-05-2016 | CN 107106023 A | | 29-08-2017 |
| | | | EP 3220813 A2 | | 27-09-2017 |
| | | | US 2016143566 A1 | | 26-05-2016 |
| | | | WO 2016081262 A2 | | 26-05-2016 |
| EP 2294978 | A1 | 16-03-2011 | EP 2294978 A1 | | 16-03-2011 |
| | | | US 2011066053 A1 | | 17-03-2011 |
| US 2014275854 | A1 | 18-09-2014 | US 2014275854 A1 | | 18-09-2014 |
| | | | US 2014276119 A1 | | 18-09-2014 |
| | | | US 2014288436 A1 | | 25-09-2014 |
| | | | US 2014288438 A1 | | 25-09-2014 |
| | | | US 2015196256 A1 | | 16-07-2015 |
| | | | US 2017027523 A1 | | 02-02-2017 |

EPO FORM P0459

For more details about this annex : see Official Journal of the European Patent Office, No. 12/82